# EUROPÄISCHE PATENTSCHRIFT

(11) **EP 4 525 780 B1**
(45) Veröffentlichungstag und Bekanntmachung des Hinweises auf die Patenterteilung: **06.08.2025**
(21) Anmeldenummer: 23723605.4
(22) Anmeldetag: 12.05.2023
(51) Int. Cl.: A61F 2/16

(54) **INTRAOKULARLINSE, DIE EINEN MEDIKAMENTENSPEICHER AUFNEHMEN KANN**
INTRAOCULAR LENS THAT CAN ACCOMMODATE A DRUG RESERVOIR
LENTILLE INTRAOCULAIRE POUVANT RESEVOIR UN RÉSERVOIR DE MÉDICAMENT

(30) Priorität: 20.05.2022 DE 102022112803
(43) Veröffentlichungstag der Anmeldung: 26.03.2025
(73) Patentinhaber: Carl Zeiss Meditec AG, 07745 Jena (DE)
(72) Erfinder: DAMM, Niklas, 73447 Oberkochen (DE); WOLFSTEIN, André, 73447 Oberkochen (DE)
(74) Vertreter: PATERIS Patentanwälte PartmbB
(86) Internationale Anmeldenummer: PCT/EP2023/062816
(87) Internationale Veröffentlichungsnummer: WO 2023/222551

(56) Entgegenhaltungen:
- WO-A1-2020/264425
- WO-A1-2021/247846
- WO-A1-2022/076456

## Beschreibung

Die Erfindung betrifft eine Intraokularlinse die einen Medikamentspeicher aufnehmen kann. WO 2021/247846 A1 offenbart ein ophthalmisches Implantationssystem für eine Medikamentenabgabe. WO 2020/264425 A1 offenbart eine okulare Verabreichungsvorrichtung.

Bei einer Kataraktbehandlung eines Auges wird eine natürliche Linse durch eine künstliche Intraokularlinse ersetzt. Dazu wird herkömmlich nur ein kleiner Schnitt in die Hornhaut des Auges eingebracht, der so groß ist, dass eine Spitze eines Injektors durch den Schnitt in das Auge eingeführt werden kann. Nachdem der Schnitt in die Hornhaut eingebracht wurde, wird die natürliche Linse des Auges üblicherweise mittels Phakoemulsifikation zerkleinert und anschließend aus dem Kapselsack des Auges abgesaugt. Danach wird die Intraokularlinse mittels des Injektors in das Auge eingesetzt. Nach der Kataraktbehandlung werden herkömmlich Medikamente, wie beispielsweise Antibiotika und/oder Entzündungshemmer, in das Auge eingebracht. Beispielsweise kann an der Intraokularlinse ein Medikamentenspeicher angebracht sein, der zusammen mit der Intraokularlinse in das Auge eingebracht wird. Wenn die Intraokularlinse mittels des Injektors in das Auge eingesetzt wird, sollte dabei der Medikamentenspeicher sich nicht von der Intraokularlinse lösen. Auch sollte der Medikamentenspeicher nicht derart sperrig ausgeführt sein, dass der Medikamentenspeicher zu einer nicht zentrischen Anordnung eines Optikkörpers der Intraokularlinse in dem Auge führt oder Strukturen zur Verhinderung eines Nachstars nicht negativ beeinflusst werden. US 2022 / 0 104 936 A1 offenbart ein ophthalmisches Implantat mit einer Intraokularlinse und Haptiken sowie Medikamentenlieferungsvorrichtungen, die an den Haptiken befestigt sind. US 2020 / 0 405 538 A1 offenbart eine ophthalmische Vorrichtung, die ein aktives oder diagnostisches Mittel aufweisen kann.

Aufgabe der Erfindung ist es daher, eine Intraokularlinse zu schaffen, die einen Medikamentenspeicher aufnehmen kann, wobei der Medikamentenspeicher, wenn er von der Intraokularlinse aufgenommen ist, sich nicht von der Intraokularlinse löst und nicht zu einer Dezentrierung eines Optikkörpers der Intraokularlinse in einem Auge führt.

Die erfindungsgemäße Intraokularlinse weist einen Optikkörper, der eine optische Achse aufweist und einen Haptikarm auf, der an dem Optikkörper befestigt ist, wobei der Haptikarm eine Radialaussparung aufweist, die in einer Seite des Haptikarms eingebracht ist, die in einer auf die optische Achse bezogene Radialrichtung außen angeordnet ist. Durch die Radialaussparung ist die Intraokularlinse eingerichtet, einen Medikamentenspeicher aufzunehmen. Wenn der Medikamentenspeicher in der Radialaussparung angeordnet ist, ist der Medikamentenspeicher besonders fest an dem Haptikarm angeordnet. Dadurch ist eine Wahrscheinlichkeit gering, dass sich der Medikamentenspeicher von dem Haptikarm löst, insbesondere wenn die Intraokularlinse via eine Spitze eines Injektors in einen Kapselsack eines Auges injiziert wird. Dadurch, dass die Radialaussparung in der in der Radialrichtung außen liegenden Seite des Haptikarms eingebracht ist, steht der Medikamentenspeicher nicht oder nur geringfügig von dem Haptikarm in der Radialausrichtung nach außen vor. Weil die in der Radialrichtung außen liegende Seite des Haptikarms den Kapselsack kontaktiert, können somit eine Dezentrierung eines Optikkörpers der Intraokularlinse in dem Auge durch den Medikamentenspeicher verringert oder sogar vermieden werden und Strukturen zur Verhinderung eines Nachstars nicht negativ beeinflusst werden.

Es ist bevorzugt, dass die Intraokularlinse einen Medikamentenspeicher aufweist, der ein Medikament und ein Durchgangsloch aufweist, in dem der Haptikarm angeordnet ist, und der in der Radialaussparung angeordnet ist. Es ist besonders bevorzugt, dass der Medikamentenspeicher in der Radialaussparung versenkt ist. Dadurch steht der Medikamentenspeicher in der Radialrichtung nicht nach außen von dem Haptikarm vor, wodurch die Dezentrierung des Optikkörpers besonders sicher vermieden werden kann.

Der Medikamentenspeicher weist bevorzugt eine erste Stirnfläche und eine zweite Stirnfläche auf, die der ersten Stirnfläche abgewandt angeordnet ist, wobei das Durchgangsloch von der ersten Stirnfläche und der zweiten Stirnfläche begrenzt ist, in einem Bereich der ersten Stirnfläche ein erstes Längsende aufweist und in einem Bereich der zweiten Stirnfläche ein zweites Längsende aufweist, wobei der Medikamentenspeicher eine Verlagerungsrichtung aufweist, die von dem ersten Längsende zu dem zweiten Längsende gerichtet ist. Um den Medikamentenspeicher in die Radialaussparung einzubringen, kann der Haptikarm ein Längsende aufweisen, das dem Optikkörper abgewandt angeordnet ist und das in das Durchgangsloch eingebracht werden kann. Anschließend kann durch ein Verlagern des Medikamentenspeichers in der Verlagerungsrichtung der Medikamentenspeicher so lange verlagert werden bis der Medikamentenspeicher in die Radialaussparung gelangt. Es ist denkbar, dass die Verlagerungsrichtung parallel zu einer Normalen der ersten Stirnfläche und/oder parallel zu einer Normalen der zweiten Stirnfläche angeordnet ist. Die Verlagerungsrichtung liegt bevorzugt in einer Ebene, deren Normale parallel zu der optischen Achse angeordnet ist. Alternativ bevorzugt ist die Verlagerungsrichtung parallel zu der optischen Achse angeordnet.

Es ist bevorzugt, dass der Haptikarm eine erste Axialaussparung aufweist, die in einer ersten Seite des Haptikarms eingebracht ist, die in einer auf die optische Achse bezogene Axialrichtung außen angeordnet ist. Dabei ist besonders bevorzugt, dass der Medikamentenspeicher in der ersten Axialaussparung angeordnet ist. Dadurch kann der Medikamentenspeicher noch fester an dem Haptikarm angeordnet sein und ein Vorstehen des Medikamentenspeichers von dem Haptikarm kann noch weiter verringert werden, wodurch eine Wahrscheinlichkeit, dass bei einem Injizieren der Intraokularlinse sich der Medikamentenspeicher von dem Haptikarm löst, noch weiter verringert werden kann. Es ist bevorzugt, dass die erste Axialaussparung unmittelbar an die Radialaussparung angrenzt.

Der Medikamentenspeicher weist bevorzugt eine erste Medikamentenspeicheraussparung, die mit dem Durchgangsloch kommuniziert, und einen ersten Steg auf, der die erste Medikamentenspeicheraussparung begrenzt, wobei der erste Steg in der ersten Axialaussparung und der Haptikarm in der ersten Medikamentenspeicheraussparung angeordnet sind. Insbesondere kann der erste Steg in der ersten Axialaussparung versenkt sein. Dadurch steht der erste Steg vorteilhaft nicht in der Axialrichtung von dem Haptikarm vor.

Es ist bevorzugt, dass der Haptikarm eine zweite Axialaussparung aufweist, die in einer auf die optische Achse bezogene Umfangsrichtung versetzt zu der ersten Axialaussparung angeordnet ist und in einer zweiten Seite des Haptikarms eingebracht ist, die entgegen der Axialrichtung außen angeordnet ist und der ersten Seite abgewandt angeordnet ist. Dabei ist besonders bevorzugt, dass der Medikamentenspeicher in der zweiten Axialaussparung angeordnet ist. Dadurch kann der Medikamentenspeicher noch fester an dem Haptikarm angeordnet sein und ein Vorstehen des Medikamentenspeichers von dem Haptikarm kann noch weiter verringert werden, wodurch eine Wahrscheinlichkeit, dass bei einem Injizieren der Intraokularlinse sich der Medikamentenspeicher von dem Haptikarm löst, noch weiter verringert werden kann. Es ist bevorzugt, dass die zweite Axialaussparung unmittelbar an die Radialaussparung angrenzt.

Der Medikamentenspeicher weist bevorzugt eine zweite Medikamentenspeicheraussparung, die mit dem Durchgangsloch kommuniziert, und einen zweiten Steg auf, der die zweite Medikamentenspeicheraussparung begrenzt, wobei der zweite Steg in der zweiten Axialaussparung und der Haptikarm in der zweiten Medikamentenspeicheraussparung angeordnet sind. Insbesondere kann der zweite Steg in der zweiten Axialaussparung versenkt sein. Dadurch steht der zweite Steg vorteilhaft nicht entgegen der Axialrichtung von dem Haptikarm vor.

Es ist bevorzugt, dass der Medikamentenspeicher in der Radialrichtung gesehen fluchtend mit dem Haptikarm angeordnet ist. Dadurch kann ein Lösen des Medikamentenspeichers von dem Haptikarm bei dem Injizieren der Intraokularlinse besonders sicher vermieden werden. Auch ist es problemlos möglich, bei einem Falten der Intraokularlinse vor dem Injizieren der Intraokularlinse den Haptikarm auf den Optikkörper zu befördern und den Optikkörper um den Haptikarm zu falten.

Es ist bevorzugt, dass die zweite Axialaussparung in der Umfangsrichtung beabstandet von der ersten Axialaussparung angeordnet ist. Dadurch kann eine starke Schwächung des Haptikarms durch die erste Axialaussparung und die zweite Axialaussparung vermieden werden, wobei die Schwächung des Haptikarms dazu führen kann, dass der Haptikarm reißt, insbesondere wenn der Haptikarm via die Spitze des Injektors in den Kapselsack eingeführt wird.

Der Haptikarm weist erfindungsgemäß einen Radialvorsprung auf, der in der Radialrichtung nach innen von dem verbliebenen Haptikarm absteht und der in einer auf die optische Achse bezogene Umfangsrichtung in dem gleichen Bereich wie die Radialaussparung angeordnet ist. Dadurch kann eine starke Schwächung des Haptikarms vermieden werden.

Es ist bevorzugt, dass der Haptikarm in dem Bereich der Radialaussparung eine Zugfestigkeit von mindestens 0,25 N hat.

Es ist bevorzugt, dass der Haptikarm gekrümmt ausgebildet ist. Besonders bevorzugt ist der Haptikarm C-förmig oder J-förmig ausgebildet.

Es ist bevorzugt, dass der Haptikarm eine weitere Radialaussparung aufweist, die in der Seite des Haptikarms eingebracht ist, die in der Radialrichtung außen angeordnet ist. Zudem kann der Haptikarm eine dritte Axialaussparung aufweisen, die in der ersten Seite des Haptikarms eingebracht ist. Außerdem ist denkbar, dass der Haptikarm eine vierte Axialaussparung aufweist, die in der zweiten Seite des Haptikarms eingebracht ist. Es ist besonders bevorzugt, dass die Intraokularlinse einen weiteren Medikamentenspeicher aufweist, der das Medikament und ein Durchgangsloch aufweist, durch das der Haptikarm sich erstreckt. Der weitere Medikamentenspeicher kann in der weiteren Radialaussparung und insbesondere in der dritten Axialaussparung und in der vierten Axialaussparung angeordnet sein.

Es ist bevorzugt, dass die Intraokularlinse einen weiteren Haptikarm aufweist, der an dem Optikkörper befestigt ist, wobei der weitere Haptikarm eine Radialaussparung aufweist, die in der Seite des Haptikarms eingebracht ist, die in einer auf die optische Achse bezogene weitere Radialrichtung außen angeordnet ist. Insbesondere kann der weitere Haptikarm punktsymmetrisch zu dem Haptikarm ausgebildet sein. Es ist besonders bevorzugt, dass die Intraokularlinse einen weiteren Medikamentenspeicher aufweist, der das Medikament und ein Durchgangsloch aufweist, durch das der weitere Haptikarm sich erstreckt. Zudem ist der weitere Medikamentenspeicher in der Radialaussparung des weiteren Haptikarms angeordnet.

Der Medikamentenspeicher ist bevorzugt mittels einer Presspassung an dem Haptikarm befestigt. Dadurch ist der Medikamentenspeicher besonders fest an dem Haptikarm angeordnet.

Es ist bevorzugt, dass der Medikamentenspeicher eingerichtet ist, das Medikament kontinuierlich abzugeben. Insbesondere kann der Medikamentenspeicher eingerichtet sein, dabei biologisch abgebaut zu werden. Dazu kann der Medikamentenspeicher eine Matrix, in das das Medikament eingebracht sein kann, mit einem Copolymer aufweisen, das von einem ersten Monomer und einem zweiten Monomer gebildet ist. Das erste Monomer kann ein Caprolacton sein und das zweite Monomer kann ausgewählt sein aus der Gruppe Laktid, Glykolid und/oder Trimethylencarbonat. Ein Beispiel für eine Matrix, in die das Medikament eingebracht sein kann und die nicht biologisch abbaubar ist, ist ein polymerisiertes Hydroxyethylmethacrylat.

Das Medikament kann beispielsweise ein Antibiotikum, wie beispielsweise Moxifloxacin, und/oder einen steroidalen Entzündungshemmer wie beispielsweise Dexamethason, und/oder einen nichtsteroidalen Entzündungshemmer, wie beispielsweise ein nichtsteroidales Antirheumatikum wie zum Beispiel Diclofenac, aufweisen. Das Medikament kann beispielsweise zusätzlich oder alternativ eine diagnostische Substanz, wie beispielsweise ein Kontrastmittel, aufweisen.

Der Medikamentenspeicher kann beispielsweise einen einzigen Werkstoff aufweisen, der die Matrix mit dem in der Matrix eingebrachten Medikament aufweist oder aus der Matrix mit dem in der Matrix eingebrachten Medikament besteht.

Der Medikamentenspeicher weist bevorzugt einen ersten Werkstoff, der die Matrix mit dem in der Matrix eingebracht Medikament aufweist, und einen zweiten Werkstoff auf, der verschieden von dem ersten Werkstoff ist sowie den ersten Steg und insbesondere den zweiten Steg bildet. Der zweite Werkstoff ist bevorzugt nicht biologisch abbaubar. Dazu kann der zweite Werkstoff ausgewählt sein aus der Gruppe Polymethylmethacrylat, polymerisiertes Hydroxyethylmethacrylat, Polypropylen, Silikon, Acrylat-Copolymer.

Im Folgenden wird anhand der beigefügten schematischen Zeichnungen die Erfindung näher erläutert. Es zeigen
Figur 1 eine Draufsicht auf eine erste erfindungsgemäße und in einem Kapselsack angeordnet gezeichnete Intraokularlinse,
Figur 2 eine perspektivische Ansicht eines ersten Medikamentenspeichers,
Figur 3 eine perspektivische Ansicht eines zweiten Medikamentenspeichers,
Figur 4 eine perspektivische Ansicht eines dritten Medikamentenspeichers,
Figur 5 eine Seitenansicht auf eine zweite erfindungsgemäße Intraokularlinse,
Figur 6 einen Haptikarm und einen Medikamentenspeicher, der beabstandet von dem Haptikarm angeordnet ist,
Figur 7 den Haptikarm und den Medikamentenspeicher aus Figur 6, wobei der Medikamentenspeicher auf den Haptikarm verlagert ist, und
Figur 8 den Haptikarm und den Medikamentenspeicher aus Figuren 6 und 7, die in einem Kapselsack angeordnet gezeichnet sind, wobei ein erster Steg des Haptikarms in einer ersten Axialaussparung des Haptikarms und ein zweiter Steg des Haptikarms in einer zweiten Axialaussparung des Haptikarms angeordnet sind.

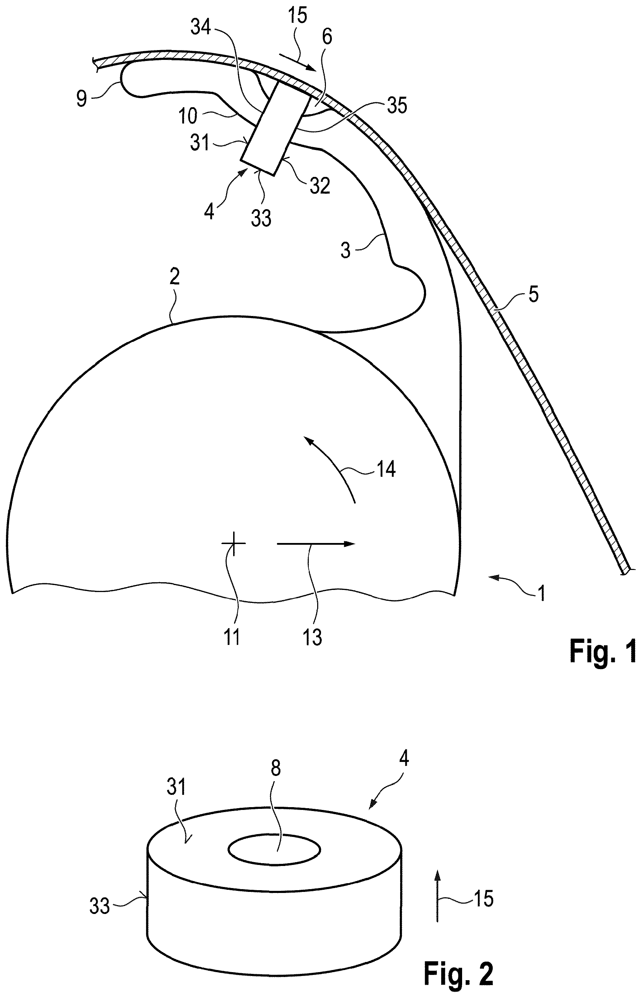

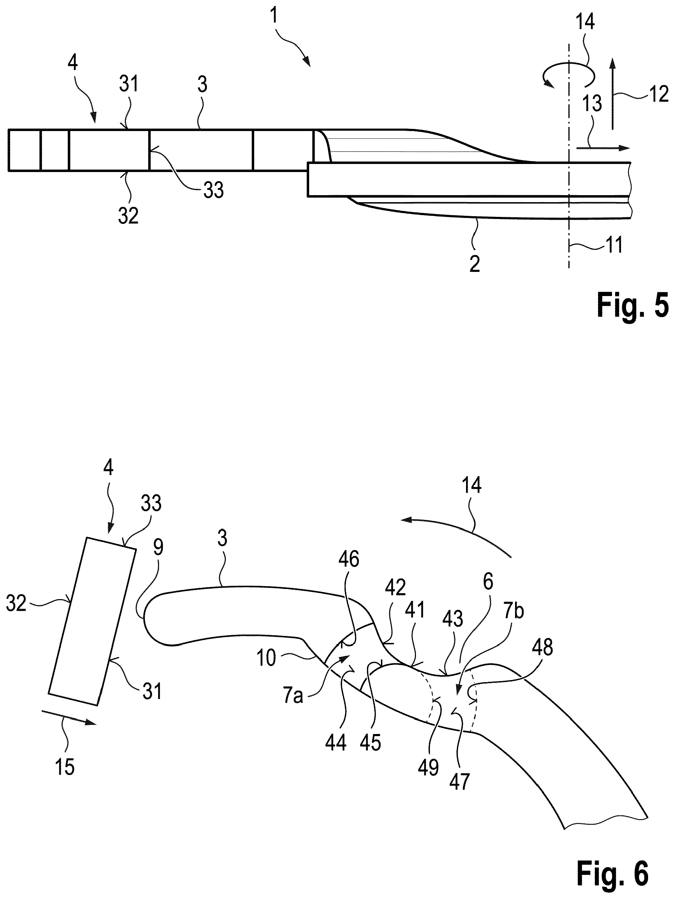

Wie es aus Figuren 1 und 5 ersichtlich ist, weist eine Intraokularlinse 1 einen Optikkörper 2, der eine optische Achse 11 aufweist, und einen Haptikarm 3 auf, der an dem Optikkörper 2 befestigt ist. Der Haptikarm 3 weist eine Radialaussparung 6 auf, die eingerichtet ist, einen Medikamentenspeicher 4 aufzunehmen und die in einer Seite des Haptikarms 3 eingebracht ist, die in einer auf die optische Achse 11 bezogene Radialrichtung 13 außen angeordnet ist. Die Intraokularlinse 1 kann einen Medikamentenspeicher 4 aufweisen, der ein Medikament aufweist und in der Radialaussparung 6 angeordnet ist. Der Medikamentenspeicher 4 weist ein Durchgangsloch 8 auf, durch das der Haptikarm 3 sich erstreckt. Der Medikamentenspeicher 4 kann beispielsweise mittels eines Formschlusses und insbesondere mittels einer Presspassung an dem Haptikarm 3 befestigt sein. Der Haptikarm 3 kann beispielsweise gekrümmt ausgebildet sein und insbesondere C-förmig (vergleiche Figur 1) oder J-förmig ausgebildet sein.

Figuren 1 bis 8 zeigen, dass der Medikamentenspeicher 4 eine erste Stirnfläche 31 und eine zweite Stirnfläche 32 aufweisen kann, die der ersten Stirnfläche 31 abgewandt angeordnet ist, wobei das Durchgangsloch 8 von der ersten Stirnfläche 31 und der zweiten Stirnfläche 32 begrenzt ist. Das Durchgangsloch 8 weist in einem Bereich der ersten Stirnfläche 31 ein erstes Längsende 34 auf und in einem Bereich der zweiten Stirnfläche 32 ein zweites Längsende 35 auf, wobei der Medikamentenspeicher 4 eine Verlagerungsrichtung 15 aufweist, die von dem ersten Längsende 34 zu dem zweiten Längsende 35 gerichtet ist. Die Verlagerungsrichtung 15 kann in die Richtung orientiert sein, in der der Medikamentenspeicher 4 zu verlagern ist, um den Medikamentenspeicher 4 in die Radialaussparung 6 zu verlagern. Es ist denkbar, dass die Verlagerungsrichtung 15 parallel zu einer Normalen der ersten Stirnfläche 31 und/oder parallel zu einer Normalen der zweiten Stirnfläche 32 angeordnet ist. Die erste Stirnfläche 31 kann das in der Verlagerungsrichtung 15 gelegene Längsende des Medikamentenspeichers 4 bilden und die zweite Stirnfläche 32 kann das in einer Richtung, die der Verlagerungsrichtung 15 entgegen gerichtet ist, gelegene Längsende des Medikamentenspeichers 4 bilden. Zudem kann der Medikamentenspeicher 4 an seiner Außenseite eine Umfangsfläche 33 aufweisen, die zwischen der ersten Stirnfläche 31 und der zweiten Stirnfläche 32 angeordnet ist und insbesondere unmittelbar an die erste Stirnfläche 31 und die zweite Stirnfläche 32 angrenzen kann.

Figur 6 zeigt, dass die Radialaussparung 6 von einer ersten Flanke 41 begrenzt sein kann, die ein Verlagern des Medikamentenspeichers 4 entgegen die Radialrichtung 13, d.h. zu der optischen Achse 11 hin, begrenzt. Zudem kann die Radialaussparung 6 von einer zweiten Flanke 42 begrenzt sein, die ein Verlagern des Medikamentenspeichers 4 in einer Umfangsrichtung 14 bezüglich der optischen Achse 11 begrenzt. Außerdem kann die Radialaussparung 6 von einer dritten Flanke 43 begrenzt sein, die ein Verlagern des Medikamentenspeichers 4 in einer Richtung, die entgegen der Umfangsrichtung 14 orientiert ist, begrenzt.

Figur 2 zeigt eine erste Ausführungsform für den Medikamentenspeicher 4, bei der das Durchgangsloch 8 zumindest in einem Punkt entlang der Verlagerungsrichtung 15 vollumfänglich von dem Werkstoff des Medikamentenspeichers 4 begrenzt ist. Es ist auch denkbar, dass das Durchgangsloch 8 in jedem Punkt entlang der Verlagerungsrichtung 15 vollumfänglich von dem Werkstoff des Medikamentenspeichers 4 begrenzt ist. Zudem zeigt Figur 2, dass das Durchgangloch 8 in einer Querschnittsebene, deren Normale parallel zu der Verlagerungsrichtung 15 ist, die Form eines Kreises haben kann. Jedoch sind auch andere Formen denkbar, wie beispielsweise eine Ellipse, ein Rechteck oder ein Quadrat. Außerdem zeigt Figur 2, dass die Umfangsfläche 33 in der Querschnittsebene die Form eines Kreises haben kann. Jedoch sind auch andere Formen denkbar, wie beispielsweise eine Ellipse, ein Rechteck oder ein Quadrat. Bei der ersten Ausführungsform des Medikamentenspeichers 4, vergleiche Figur 2, ist es denkbar, dass, wenn der Medikamentenspeicher 4 in der Radialaussparung 6 angeordnet ist, die Verlagerungsrichtung 15 in einer Ebene liegt, deren Normale parallel zu der optischen Achse 11 angeordnet ist, vergleiche Figur 1. Bei der ersten Ausführungsform des Medikamentenspeichers 4 ist es zudem denkbar, dass der Haptikarm 3 sich von dem ersten Längsende 34 bis zu dem zweiten Längsende 35 erstreckt.

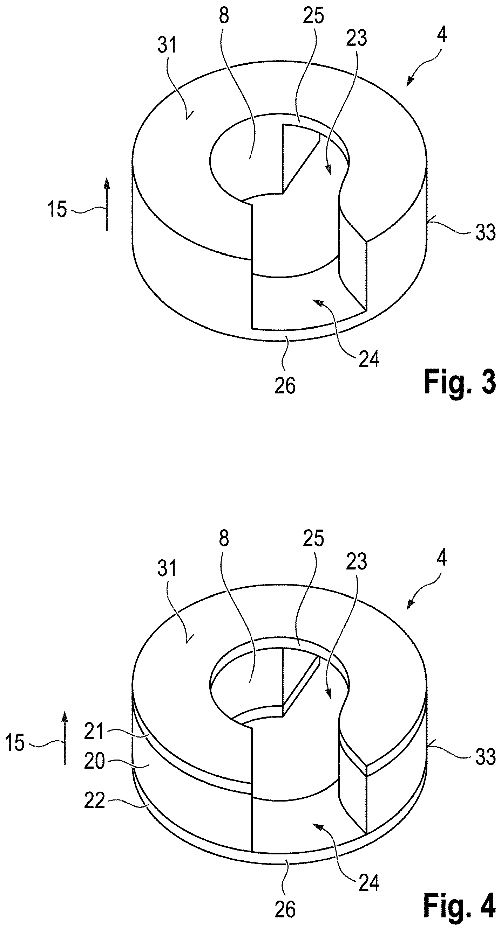

Figuren 6 bis 8 zeigen, dass der Haptikarm 3 eine erste Axialaussparung 7a aufweisen kann, in der der Medikamentenspeicher 4 angeordnet ist und die in einer ersten Seite des Haptikarms 3 eingebracht ist, die in einer auf die optische Achse 11 bezogene Axialrichtung 12 außen angeordnet ist. Dabei kann der Medikamentenspeicher 4 eine erste Medikamentenspeicheraussparung 23 (vergleiche die zweite Ausführungsform des Medikamentenspeichers 4 in Figur 3 und die dritte Ausführungsform des Medikamentenspeichers 4 in Figur 4), die mit dem Durchgangsloch 8 kommuniziert, und einen ersten Steg 25 aufweisen, der die erste Medikamentenspeicheraussparung 23 begrenzt, wobei der erste Steg 25 in der ersten Axialaussparung 7a und der Haptikarm 3 in der ersten Medikamentenspeicheraussparung 23 angeordnet sind. Der erste Steg 25 kann einen Teil der ersten Stirnfläche 31 bilden und die zweite Stirnfläche 32 kann in einem Bereich der ersten Medikamentenspeicheraussparung 23 unausgebildet sein, um ein Einführen des Haptikarms 3 in der Verlagerungsrichtung 15 in die erste Medikamentenspeicheraussparung 23 zu ermöglichen.

Aus Figuren 6 bis 8 ist zudem ersichtlich, dass der Haptikarm 3 eine zweite Axialaussparung 7b aufweisen kann, in der der Medikamentenspeicher 4 angeordnet ist und die in einer auf die optische Achse 11 bezogene Umfangsrichtung 14 versetzt zu der ersten Axialaussparung 7a angeordnet ist sowie die in einer zweiten Seite des Haptikarms 3 eingebracht ist, die entgegen der Axialrichtung 12 außen angeordnet ist und der ersten Seite abgewandt angeordnet ist. Dabei kann der Medikamentenspeicher 4 eine zweite Medikamentenspeicheraussparung 24 (vergleiche die zweite Ausführungsform des Medikamentenspeichers 4 in Figur 3 und die dritte Ausführungsform des Medikamentenspeichers 4 in Figur 4), die mit dem Durchgangsloch 8 kommuniziert, und einen zweiten Steg 26 aufweisen, der die zweite Medikamentenspeicheraussparung 24 begrenzt, wobei der zweite Steg 26 in der zweiten Axialaussparung 7b und der Haptikarm 3 in der zweiten Medikamentenspeicheraussparung 24 angeordnet sind. Der zweite Steg 26 kann einen Teil der zweiten Stirnfläche 32 bilden und die erste Stirnfläche 31 kann in einem Bereich der zweiten Medikamentenspeicheraussparung 24 unausgebildet sein, um ein Einführen des Haptikarms 3 entgegen der Verlagerungsrichtung 15 in die zweite Medikamentenspeicheraussparung 24 zu ermöglichen.

Die erste Axialaussparung 7a kann unmittelbar an die Radialaussparung 6 angrenzen und/oder die zweite Axialaussparung 7b kann unmittelbar an die Radialaussparung 6 angrenzen, vergleiche Figuren 6 bis 8. Die zweite Axialaussparung 7b kann in der Umfangsrichtung 14 beabstandet von der ersten Axialaussparung 7a angeordnet sein. Figuren 1 und 6 bis 8 zeigen, dass die Intraokularlinse 1 einen Radialvorsprung 10 aufweisen kann, der in der Radialrichtung 13 nach innen von dem verbliebenen Haptikarm 3 absteht und der in einer auf die optische Achse 11 bezogene Umfangsrichtung 14 in dem gleichen Bereich wie die Radialaussparung 6 angeordnet ist.

Der erste Steg 25 kann in der ersten Axialaussparung 7a versenkt sein, so dass der Medikamentenspeicher 4 in der Radialrichtung 13 gesehen in einem Bereich der ersten Seite fluchtend mit dem Haptikarm 3 angeordnet sein kann, vergleiche Figur 5. Der zweite Steg 26 kann in der zweiten Axialaussparung 7b versenkt sein, so dass der Medikamentenspeicher 4 in der Radialrichtung 13 gesehen in einem Bereich der zweiten Seite fluchtend mit dem Haptikarm 3 angeordnet sein kann, vergleiche Figur 5.

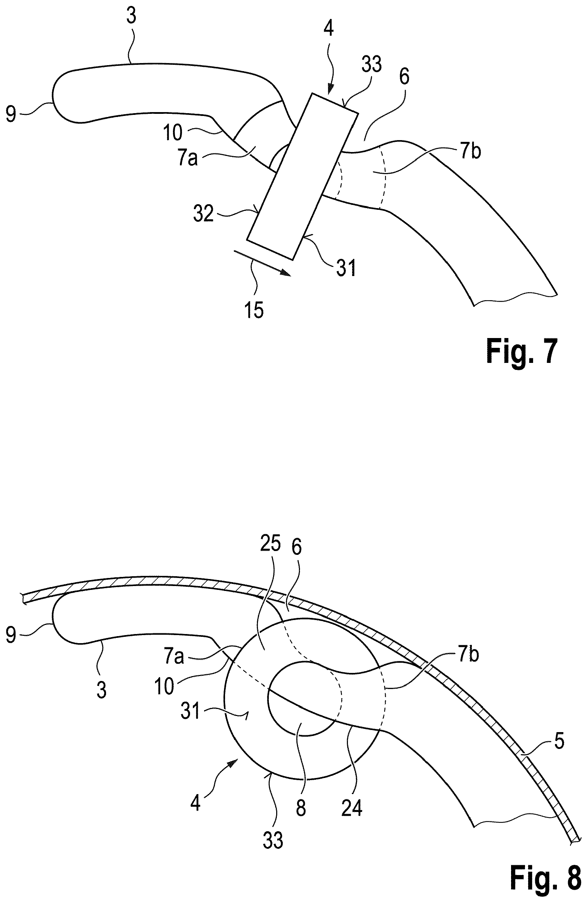

Es ist denkbar, dass für die zweite Ausführungsform des Medikamentenspeichers 4 und die dritte Ausführungsform des Medikamentenspeichers 4 die Verlagerungsrichtung 15 parallel zu der optischen Achse 11 angeordnet ist, siehe Figur 8.

Figuren 6 bis 8 zeigen, wie der Medikamentenspeicher 4 in die Radialaussparung 6 eingebracht werden kann. Zuerst ist der Medikamentenspeicher 4 beabstandet von dem Haptikarm 3 angeordnet. Anschließend kann zuerst ein Längsende 9 des Haptikarms 3, das dem Optikkörper 2 abgewandt angeordnet ist, in dem Durchgangsloch 8 angeordnet werden. Durch ein Verlagern des Medikamentenspeichers 4 in der Verlagerungsrichtung 15 kann der Medikamentenspeicher 4 so lange verlagert werden, bis der Medikamentenspeicher 4 in die Radialaussparung 6 gelangt, vergleiche Figur 7. Bei der ersten Ausführungsform des Medikamentenspeichers 4 ist hier die Verlagerung abgeschlossen und es liegt beispielsweise die in Figur 1 gezeigte Anordnung vor. Bei der zweiten und dritten Ausführungsform des Medikamentenspeichers 4 ist noch ein Schwenken des Medikamentenspeichers 4 erforderlich, so dass der erste Steg 25 in die erste Axialaussparung 7a gelangt und der zweite Steg 26 in die zweite Axialaussparung 7b gelangt, vergleiche Figur 8.

Figur 6 zeigt, dass die erste Axialaussparung 7a von einer ersten Flanke 44 begrenzt sein kann, die ein Verlagern des Medikamentenspeichers 4 entgegen der Axialrichtung 12 begrenzt. Zudem kann die erste Axialaussparung 7a von einer zweiten Flanke 45 begrenzt sein, die ein Verlagern des Medikamentenspeichers 4 entgegen der Umfangsrichtung 14 begrenzt. Außerdem kann die erste Axialaussparung 7a von einer dritten Flanke 46 begrenzt sein, die ein Verlagern des Medikamentenspeichers 4 in der Umfangsrichtung 14 begrenzt. Zudem zeigt Figur 6, dass die zweite Axialaussparung 7b von einer ersten Flanke 47 begrenzt sein kann, die ein Verlagern des Medikamentenspeichers 4 in der Axialrichtung 12 begrenzt. Zudem kann die zweite Axialaussparung 7b von einer zweiten Flanke 48 begrenzt sein, die ein Verlagern des Medikamentenspeichers 4 entgegen der Umfangsrichtung 14 begrenzt. Außerdem kann die zweite Axialaussparung 7b von einer dritten Flanke 49 begrenzt sein, die ein Verlagern des Medikamentenspeichers 4 in der Umfangsrichtung 14 begrenzt.

Die erste Flanke 41 der Radialaussparung 6 und die erste Flanke 44 der ersten Axialaussparung 7a können beispielsweise einen Winkel von 60° bis 120°, insbesondere von 80° bis 100° oder im Wesentlichen 90°, einschließen. Die erste Flanke 41 der Radialaussparung 6 und die erste Flanke 47 der zweiten Axialaussparung 7b können beispielsweise einen Winkel von 60° bis 120°, insbesondere von 80° bis 100° oder im Wesentlichen 90°, einschließen.

Der Medikamentenspeicher 4 kann nur einen einzigen Werkstoff aufweisen, der das Medikament aufweist, wie es bei der zweiten Ausführungsform des Medikamentenspeichers 4 gemäß Figur 3 der Fall ist.

Alternativ dazu ist denkbar, dass der Medikamentenspeicher 4 einen ersten Werkstoff aufweist, der das Medikament aufweist, und einen zweiten Werkstoff aufweist, der verschieden von dem ersten Werkstoff ist und den ersten Steg 25 bildet, wie es bei der dritten Ausführungsform des Medikamentenspeichers 4 gemäß Figur 4 der Fall ist. Dazu kann der Medikamentenspeicher 4 eine erste Deckplatte 21 aufweisen, die von dem zweiten Werkstoff gebildet ist und die erste Stirnfläche 31 bildet. Es ist auch denkbar, dass der zweite Werkstoff den zweiten Steg 26 bildet. Dazu kann der Medikamentenspeicher 4 eine zweite Deckplatte 22 aufweisen, die von dem zweiten Werkstoff gebildet ist und die zweite Stirnfläche 32 bildet. Zwischen der ersten Deckplatte 21 und der zweiten Deckplatte 22 kann ein Mittelteil 20 angeordnet sein, das von dem ersten Werkstoff gebildet ist und insbesondere die Umfangsfläche 33 bildet.

Die erste Deckplatte 21 und/oder die zweite Deckplatte 22 können porös sein. Dadurch ist es möglich, dass das Medikament schneller freigesetzt wird.

Der Medikamentenspeicher 4 kann eine Säule aufweisen, die sich durch den ersten Werkstoff erstrecken und die die erste Deckplatte 21 und die zweite Deckplatte 22 miteinander verbindet. Die Säule kann beispielsweise den zweiten Werkstoff aufweisen und/oder aus dem zweiten Werkstoff bestehen. Es ist auch denkbar, dass mehrere der Säulen vorgesehen sind.

Figuren 1 und 8 zeigen, dass der Medikamentenspeicher 4 in der Radialaussparung 6 versenkt sein kann. Dadurch wird erreicht, dass der Medikamentenspeicher 4, wenn die Intraokularlinse 1 in den Kapselsack 5 eines Auges eingebracht ist, den Kapselsack 5 nicht nach außen, d.h. weg von dem Optikkörper 2, ausbeult, vergleiche Figuren 1 und 8.

### Bezugszeichenliste

- 1: Intraokularlinse
- 2: Optikkörper
- 3: Haptikarm
- 4: Medikamentenspeicher
- 5: Kapselsack
- 6: Radialaussparung
- 7a: erste Axialaussparung
- 7b: zweite Axialaussparung
- 8: Durchgangsloch
- 9: Längsende
- 10: Radialvorsprung
- 11: optische Achse
- 12: Axialrichtung
- 13: Radialrichtung
- 14: Umfangsrichtung
- 15: Verlagerungsrichtung
- 20: Mittelteil
- 21: erste Deckplatte
- 22: zweite Deckplatte
- 23: erste Medikamentenspeicheraussparung
- 24: zweite Medikamentenspeicheraussparung
- 25: erster Steg
- 26: zweiter Steg
- 31: erste Stirnfläche
- 32: zweite Stirnfläche
- 33: Umfangsfläche
- 34: erstes Längsende
- 35: zweites Längsende
- 41: erste Flanke der Radialaussparung
- 42: zweite Flanke der Radialaussparung
- 43: dritte Flanke der Radialaussparung
- 44: erste Flanke der ersten Axialaussparung
- 45: zweite Flanke der ersten Axialaussparung
- 46: dritte Flanke der ersten Axialaussparung
- 47: erste Flanke der zweiten Axialaussparung
- 48: zweite Flanke der zweiten Axialaussparung
- 49: dritte Flanke der dritten Axialaussparung

## Patentansprüche

1. Intraokularlinse mit einem Optikkörper (2), der eine optische Achse (11) aufweist, und einem Haptikarm (3), der an dem Optikkörper (2) befestigt ist, wobei der Haptikarm (3) eine Radialaussparung (6) aufweist, die in einer Seite des Haptikarms (3) eingebracht ist, die in einer auf die optische Achse (11) bezogene Radialrichtung (13) außen angeordnet ist, **dadurch gekennzeichnet, dass** der Haptikarm (3) einen Radialvorsprung (10) aufweist, der in der Radialrichtung (13) nach innen von dem verbliebenen Haptikarm (3) absteht und der in einer auf die optische Achse (11) bezogene Umfangsrichtung (14) in dem gleichen Bereich wie die Radialaussparung (6) angeordnet ist.

2. Intraokularlinse gemäß Anspruch 1, wobei die Intraokularlinse (1) einen Medikamentenspeicher (4) aufweist, der ein Medikament und ein Durchgangsloch (8) aufweist, in dem der Haptikarm (3) angeordnet ist, und der in der Radialaussparung (6) angeordnet ist.

3. Intraokularlinse gemäß Anspruch 2, wobei der Medikamentenspeicher (4) in der Radialaussparung (6) versenkt ist.

4. Intraokularlinse gemäß einem der Ansprüche 1 bis 3, wobei der Haptikarm (3) eine erste Axialaussparung (7a) aufweist, die in einer ersten Seite des Haptikarms (3) eingebracht ist, die in einer auf die optische Achse (11) bezogene Axialrichtung (12) außen angeordnet ist, insbesondere wobei der Medikamentenspeicher (4) in der ersten Axialaussparung (7a) angeordnet ist.

5. Intraokularlinse gemäß Anspruch 4, wobei die erste Axialaussparung (7a) unmittelbar an die Radialaussparung (6) angrenzt.

6. Intraokularlinse Anspruch 4 oder 5, wobei der Haptikarm (3) eine zweite Axialaussparung (7b) aufweist, die in einer auf die optische Achse (11) bezogene Umfangsrichtung (14) versetzt zu der ersten Axialaussparung (7a) angeordnet ist und in einer zweiten Seite des Haptikarms (3) eingebracht ist, die entgegen der Axialrichtung (12) außen angeordnet ist und der ersten Seite abgewandt angeordnet ist, insbesondere wobei der Medikamentenspeicher (4) in der zweiten Axialaussparung (7b) angeordnet ist.

7. Intraokularlinse gemäß Anspruch 6, wobei die zweite Axialaussparung (7b) unmittelbar an die Radialaussparung (6) angrenzt.

8. Intraokularlinse gemäß Anspruch 6 oder 7, wobei die zweite Axialaussparung (7b) in der Umfangsrichtung (14) beabstandet von der ersten Axialaussparung (7a) angeordnet ist.

9. Intraokularlinse gemäß einem der Ansprüche 1 bis 8, wobei der Haptikarm (3) gekrümmt ausgebildet ist und insbesondere C-förmig oder J-förmig ausgebildet ist.

## Claims

1. Intraocular lens comprising an optical body (2), which has an optical axis (11), and a haptic arm (3), which is attached to the optical body (2), wherein the haptic arm (3) has a radial cutout (6) formed in a side of the haptic arm (3), which side is arranged outwards in a radial direction (13) with respect to the optical axis (11), **characterized in that** the haptic arm (3) has a radial projection (10) which projects inwards in the radial direction (13) from the rest of the haptic arm (3) and which, in a circumferential direction (14) with respect to the optical axis (11), is arranged in the same region as the radial cutout (6).

2. Intraocular lens according to Claim 1, wherein the intraocular lens (1) has a medicament reservoir (4) which has a medicament and a through-hole (8), in which the haptic arm (3) is arranged, and which is arranged in the radial cutout (6).

3. Intraocular lens according to Claim 2, wherein the medicament reservoir (4) is recessed in the radial cutout (6).

4. Intraocular lens according to any one of Claims 1 to 3, wherein the haptic arm (3) has a first axial cutout (7a), which is formed in a first side of the haptic arm (3), which side is arranged outwards in an axial direction (12) with respect to the optical axis (11), in particular wherein the medicament reservoir (4) is arranged in the first axial cutout (7a).

5. Intraocular lens according to Claim 4, wherein the first axial cutout (7a) directly adjoins the radial cutout (6).

6. Intraocular lens according to Claim 4 or 5, wherein the haptic arm (3) has a second axial cutout (7b), which is arranged offset from the first axial cutout (7a) in a circumferential direction (14) with respect to the optical axis (11) and is formed in a second side of the haptic arm (3), which side is arranged outwards counter to the axial direction (12) and is arranged facing away from the first side, in particular wherein the medicament reservoir (4) is arranged in the second axial cutout (7b).

7. Intraocular lens according to Claim 6, wherein the second axial cutout (7b) directly adjoins the radial cutout (6).

8. Intraocular lens according to Claim 6 or 7, wherein the second axial cutout (7b) is arranged spaced apart from the first axial cutout (7a) in the circumferential direction (14).

9. Intraocular lens according to any one of Claims 1 to 8, wherein the haptic arm (3) has a curved design and in particular has a C-shaped or J-shaped design.

## Revendications

1. Lentille intraoculaire avec un corps optique (2) présentant un axe optique (11) et un bras haptique (3) fixé au corps optique (2), le bras haptique (3) présentant un évidement radial (6) pratiqué dans un côté du bras haptique (3) qui est agencé à l'extérieur dans une direction radiale (13) par rapport à l'axe optique (11), **caractérisé en ce que** le bras haptique (3) présente une saillie radiale (10) qui fait saillie vers l'intérieur du bras haptique restant (3) dans la direction radiale (13) et qui est agencée dans la même zone que l'évidement radial (6) dans une direction circonférentielle (14) par rapport à l'axe optique (11).

2. Lentille intraoculaire selon la revendication 1, la lentille intraoculaire (1) présentant un réservoir de médicament (4) qui présente un médicament et un trou traversant (8) dans lequel le bras haptique (3) est agencé, et qui est agencé dans l'évidement radial (6).

3. Lentille intraoculaire selon la revendication 2, le réservoir de médicament (4) étant encastré dans l'évidement radial (6).

4. Lentille intraoculaire selon l'une quelconque des revendications 1 à 3, le bras haptique (3) présentant un premier évidement axial (7a) pratiqué dans un premier côté du bras haptique (3) agencé à l'extérieur dans une direction axiale (12) par rapport à l'axe optique (11), le réservoir de médicament (4) étant notamment agencé dans le premier évidement axial (7a).

5. Lentille intraoculaire selon la revendication 4, le premier évidement axial (7a) étant directement adjacent à l'évidement radial (6).

6. Lentille intraoculaire revendication 4 ou 5, le bras haptique (3) présentant un deuxième évidement axial (7b) agencé en décalage du premier évidement axial (7a) dans une direction circonférentielle (14) par rapport à l'axe optique (11) et pratiqué dans un deuxième côté du bras haptique (3) agencé à l'extérieur à l'encontre de la direction axiale (12) et agencé à l'opposé du premier côté, le réservoir de médicaments (4) étant notamment agencé dans le deuxième évidement axial (7b).

7. Lentille intraoculaire selon la revendication 6, le deuxième évidement axial (7b) étant directement adjacent à l'évidement radial (6).

8. Lentille intraoculaire selon la revendication 6 ou 7, le deuxième évidement axial (7b) étant agencé à distance du premier évidement axial (7a) dans la direction circonférentielle (14).

9. Lentille intraoculaire selon l'une quelconque des revendications 1 à 8, le bras haptique (3) étant réalisé sous forme incurvée et étant notamment réalisé en forme de C ou en forme de J.
